# EUROPEAN PATENT APPLICATION

(11) **EP 4 682 265 A1**
(43) Date of publication of application: **21.01.2026**
(21) Application number: 25189864.9
(22) Date of filing: 16.07.2025
(51) Int. Cl.: C12Q 1/18

(54) **METHOD FOR EVALUATING DRUG RESISTANCE OF MICROORGANISMS**

(30) Priority: 17.07.2024 JP 2024114225
(71) Applicant: SHIMADZU CORPORATION, Kyoto-shi, Kyoto 604-8511 (JP)
(72) Inventor: MATSUMURA, Yuriko, Shinagawa-ku, Tokyo, 141-8648 (JP); KAJI, Daiki, Shinagawa-ku, Tokyo, 141-8648 (JP); OGATA, Koretsugu, Kyoto-shi, Kyoto, 604-8511 (JP)
(74) Representative: Müller-Boré & Partner Patentanwälte PartG mbB

(57) **Abstract**

[Summary]

[Problem] To provide a method for rapidly evaluating the resistance of microorganisms such as bacteria to a drug.

[Solution] The method comprises an extraction step of performing an operation to obtain an extract containing a drug-degrading enzyme that can be produced by the microorganism from a sample containing the microorganism; a mixing step of mixing the extract and a drug to obtain a mixture; an analysis step of subjecting the mixture to mass spectrometry; and an evaluation step of determining whether the microorganism is resistant to the drug by detecting the presence or absence of a peak derived from a degradation product of the drug from the mass spectrum obtained in the analysis step.

## Description

### [TECHNICAL FIELD]

The present invention relates to a method for evaluating drug resistance of a microorganism.

### [BACKGROUND ART]

In recent years, a major problem has been the increasing proportion of bacteria that are resistant to conventional antimicrobial agents (so-called drug-resistant bacteria) due to the overuse of antimicrobial agents for patients with infectious diseases. Rapidly identifying the causative agent of an infectious disease and evaluating the resistance of the causative agent to various antimicrobial agents, followed by the early prescription of an appropriate antimicrobial agent, is important not only for the early recovery of the patient but also for suppressing the emergence of drug-resistant bacteria.

One method for identifying the causative agent of an infectious disease is a method using a matrix-assisted laser desorption/ionization mass spectrometer (MALDI-MS). In this method, first, a specimen collected from a patient is subjected to a predetermined treatment as necessary, then mixed with a matrix solution and dried, and subjected to mass spectrometry by MALDI-MS. Then, the pattern of the obtained mass spectrum is collated with the mass spectrum patterns of a large number of known bacteria previously registered in a database (including the positions and shapes of peaks characteristic of each bacterium (marker peaks)) to identify the bacteria contained in the specimen. Since the measurement by MALDI-MS is completed in just a few minutes, the causative agent of an infectious disease can be rapidly identified according to the above method.

On the other hand, as a method for evaluating the resistance of bacteria to antimicrobial agents, a method of detecting metabolites of antimicrobial agents by bacteria through mass spectrometry is known. In this method, it is common to perform mass spectrometry using a liquid chromatograph-mass spectrometer (LC-MS). However, if the mass spectrometry of metabolites can be performed using the same MALDI-MS used for identifying the causative agent as described above, the identification of the causative agent and the evaluation of the antimicrobial resistance of the causative agent can be performed with a single apparatus. Therefore, methods for evaluating the antimicrobial resistance of bacteria by performing mass spectrometry on the metabolites of antimicrobial agents using MALDI-MS are being studied.

Some types of bacteria are known to exhibit resistance to β-lactam (penicillin-based and cephalosporin-based) antimicrobial agents by having the property of producing β-lactamase, an enzyme that degrades drugs (hereinafter referred to as a drug-digested enzyme). β-lactamase hydrolyzes the β-lactam ring of β-lactam antimicrobial agents to inactivate them. For example, in the method described in Patent Literature 1, a solution consisting of a β-lactam antimicrobial agent is added to bacteria, and the bacterial solution is cultured for several hours at a predetermined temperature. Next, the bacteria are separated from the cultured bacterial solution, and a solution containing metabolites of the β-lactam antimicrobial agent that may be produced by the metabolism of the bacteria and the remaining β-lactam antimicrobial agent is subjected to mass spectrometry by MALDI-MS. In the obtained mass spectrum, if it is confirmed that the intensity of the peak corresponding to the β-lactam antimicrobial agent has decreased and a peak corresponding to the metabolite of the antimicrobial agent has appeared, it can be determined that the bacterium is resistant to the β-lactam antimicrobial agent used in the evaluation.

### [CITATION LIST]

### [PATENT LITERATURE]

[Patent Literature 1] Japanese Unexamined Patent Application Publication No. 2013-529458

### [SUMMARY OF THE INVENTION]

### [PROBLEM TO BE SOLVED BY THE INVENTION]

The method for evaluating antimicrobial resistance of bacteria described in Patent Literature 1 detects the degradation products (metabolites) of the antimicrobial agent produced by the metabolism of the bacteria. Therefore, the method described in Patent Literature 1 has a problem in that it requires culturing the bacteria in the presence of the antimicrobial agent, which takes time.

A problem to be solved by the present invention is to provide a method for rapidly evaluating the resistance of microorganisms such as bacteria to a drug.

### [MEANS FOR SOLVING THE PROBLEM]

The method for evaluating drug resistance of a microorganism according to the present invention, which has been made to solve the above problem, comprises:
an extraction step of performing an operation to obtain an extract containing a drug-digested enzyme that can be produced by the microorganism from a sample containing the microorganism;
a mixing step of mixing the extract and a drug to obtain a mixture;
an analysis step of complexing the mixture to mass spectrometry; and
an evaluation step of determining whether the microorganism is resistant to the drug by detecting the presence or absence of a peak derived from a degradation product of the drug from the mass spectrum obtained in the analysis step.

### [EFFECTS OF THE INVENTION]

According to the above configuration, since the drug-digested enzyme extracted from the microorganism degrades the drug, it is not necessary to culture the microorganism in the process where the drug is degraded. Therefore, the presence or absence of resistance of the microorganism to the drug can be evaluated rapidly.

### [BRIEF DESCRIPTION OF THE DRAWINGS]

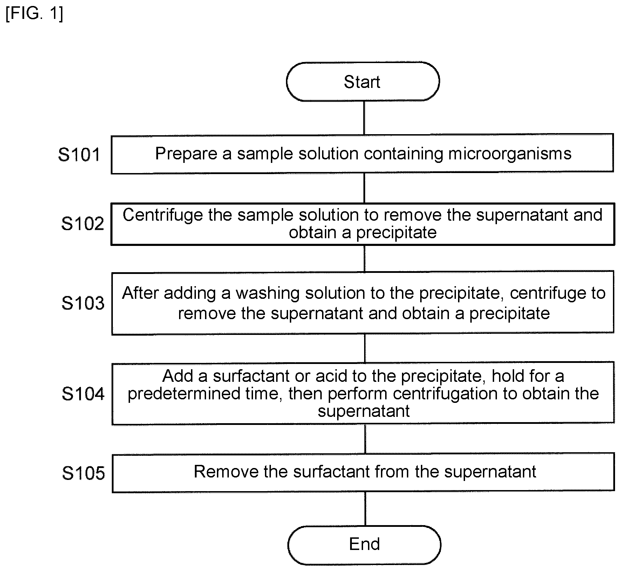

[FIG. 1] FIG. 1 is a flowchart showing the procedure of operations in the extraction step of the method for evaluating drug resistance of a microorganism according to the present embodiment.

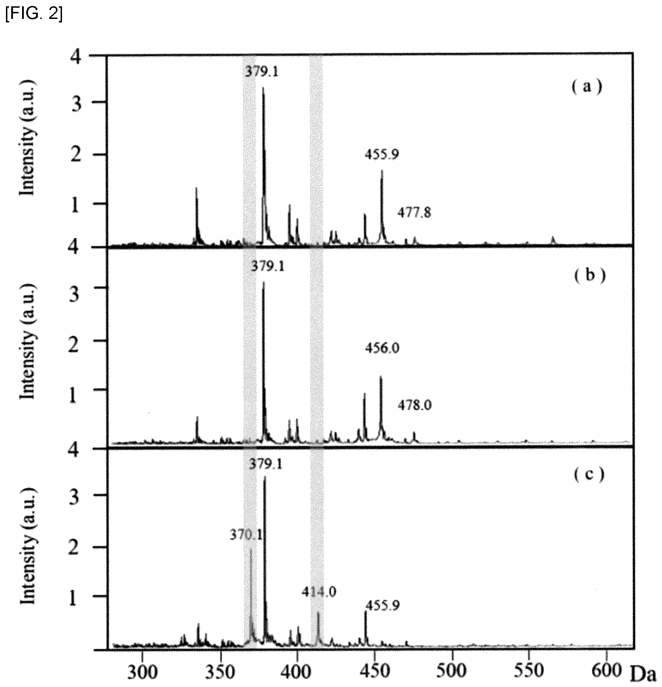

[FIG. 2] FIG. 2 is, in Experimental Example 1, the mass spectrum when saline solution was brought into contact with CTX (FIG. 2(a)), the mass spectrum when a bacterial suspension of a susceptible strain was brought into contact with CTX (FIG. 2(b)), and the mass spectrum when a bacterial suspension of an ESBL-producing strain was brought into contact with CTX (FIG. 2(c)).

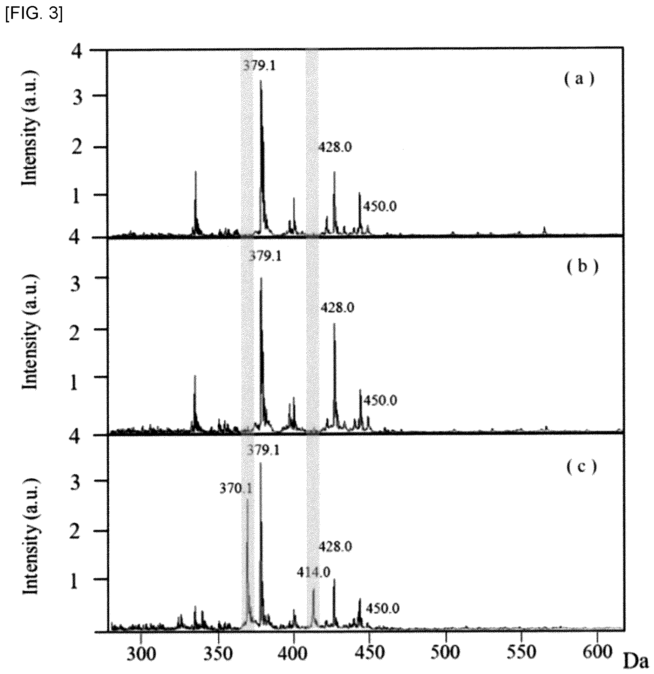

[FIG. 3] FIG. 3 is, in Experimental Example 1, the mass spectrum when saline solution was brought into contact with CPDX (FIG. 3(a)), the mass spectrum when a bacterial suspension of a susceptible strain was brought into contact with CPDX (FIG. 3(b)), and the mass spectrum when a bacterial suspension of an ESBL-producing strain was brought into contact with CPDX (FIG. 3(c)).

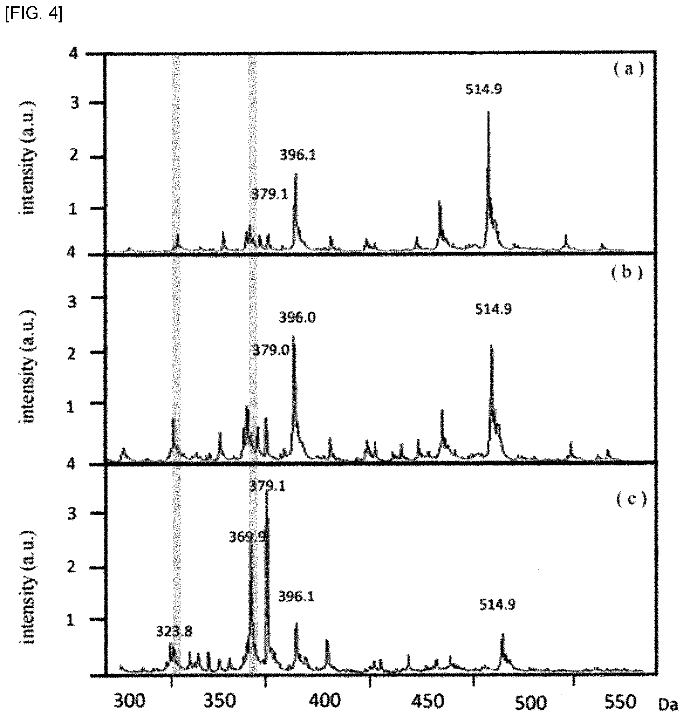

[FIG. 4] FIG. 4 is, in Example 1, the mass spectrum when saline solution was brought into contact with CPR (FIG. 4(a)), the mass spectrum when an extract from a susceptible strain was brought into contact with CPR (FIG. 4(b)), and the mass spectrum when an extract from an ESBL-producing strain was brought into contact with CPR (FIG. 4(c)).

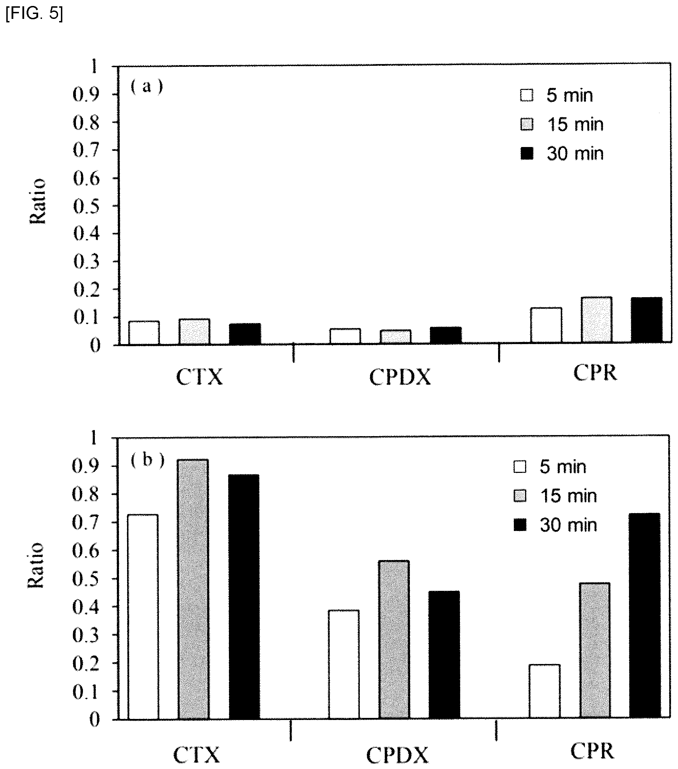

[FIG. 5] FIG. 5 is, in Example 2, a diagram showing the Ihydro/Itotal values when an extract from a susceptible strain was brought into contact with various antimicrobial agents for a predetermined time (FIG. 5(a)), and a diagram showing the Ihydro/Itotal values when an extract from an ESBL-producing strain was brought into contact with various antimicrobial agents for a predetermined time (FIG. 5(b)).

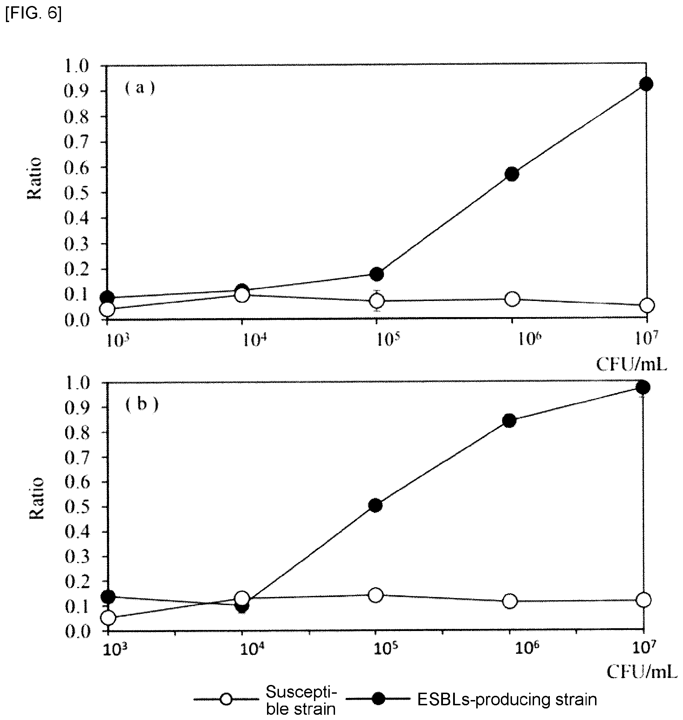

[FIG. 6] FIG. 6 is, in Example 3, a diagram showing the Ihydro/Itotal values when an extract prepared using a bacterial suspension adjusted to various bacterial concentrations was brought into contact with CTX for 15 minutes (FIG. 6(a)), and a diagram showing the Ihydro/Itotal values when the extract was brought into contact with CTX for 30 minutes (FIG. 6(b)).

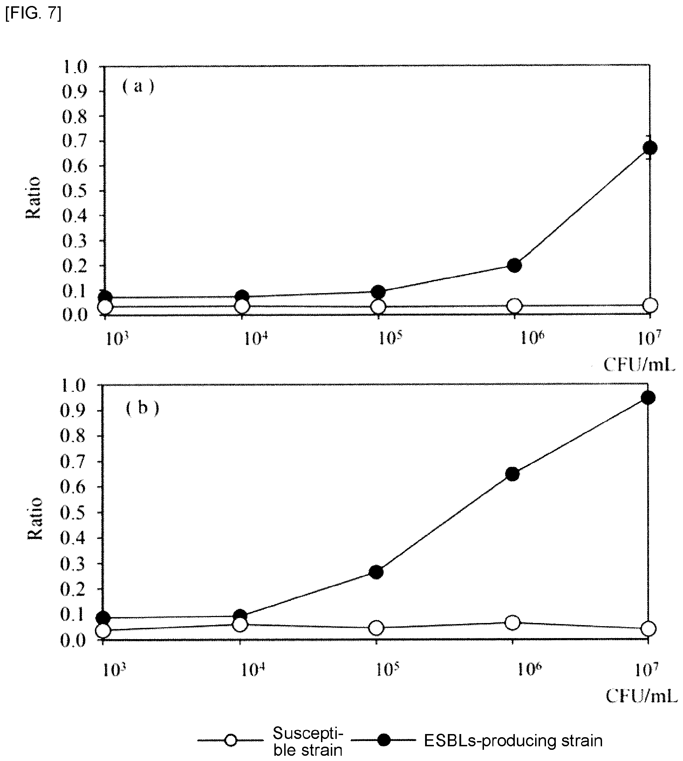

[FIG. 7] FIG. 7 is, in Example 3, a diagram showing the Ihydro/Itotal values when an extract prepared using a bacterial suspension adjusted to various bacterial concentrations was brought into contact with CPDX for 15 minutes (FIG. 7(a)), and a diagram showing the Ihydro/Itotal values when the extract was brought into contact with CPDX for 30 minutes (FIG. 7(b)).

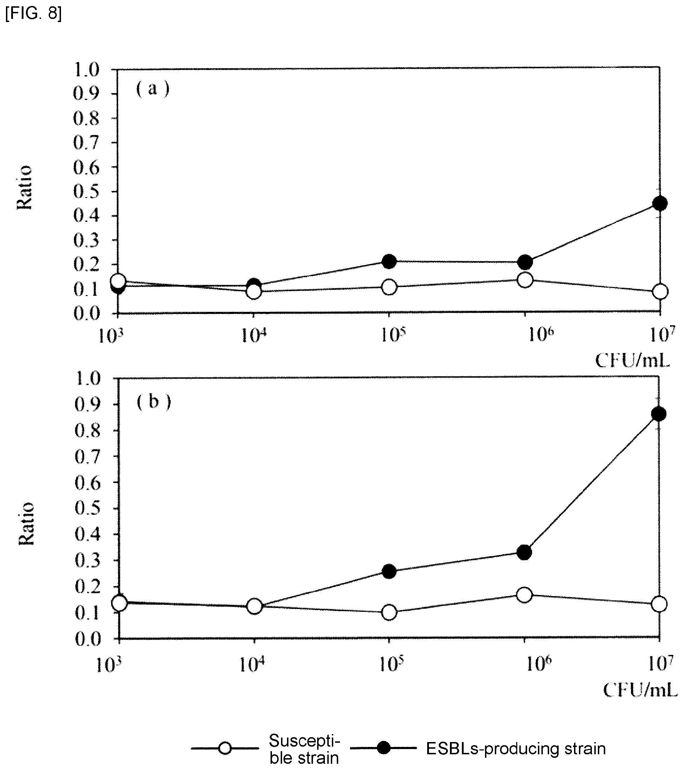

[FIG. 8] FIG. 8 is, in Example 3, a diagram showing the Ihydro/Itotal values when an extract prepared using a bacterial suspension adjusted to various bacterial concentrations was brought into contact with CPR for 15 minutes (FIG. 8(a)), and a diagram showing the Ihydro/Itotal values when the extract was brought into contact with CPR for 30 minutes (FIG. 8(b)).

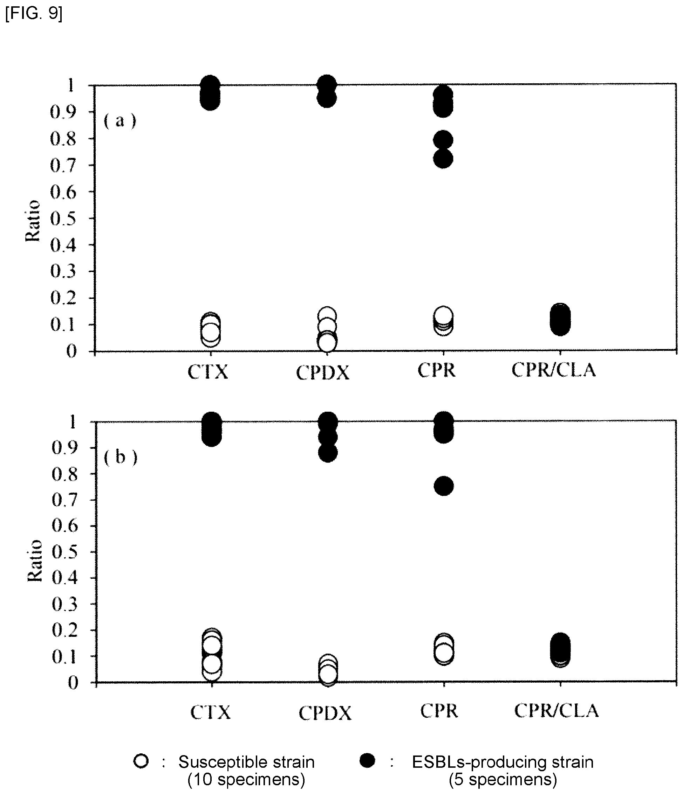

[FIG. 9] FIG. 9 is, in Example 4, a diagram showing the Ihydro/Itotal values when an extract from a urine specimen was brought into contact with various antimicrobial agents for 15 minutes (FIG. 9(a)), and a diagram showing the Ihydro/Itotal values when an extract from a urine specimen was brought into contact with various antimicrobial agents for 30 minutes (FIG. 9(b)).

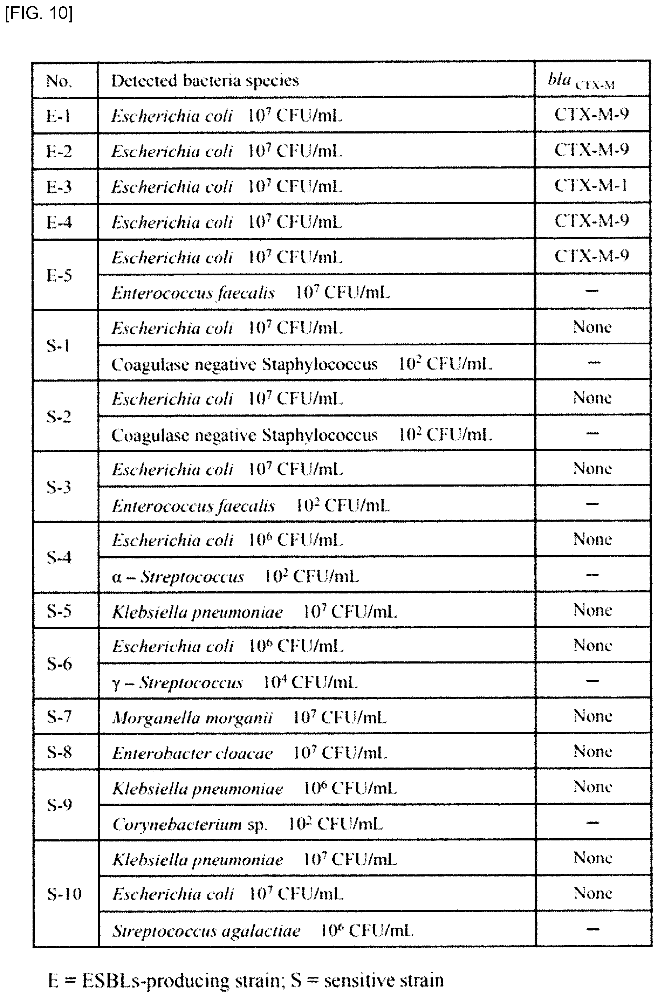

[FIG. 10] FIG. 10 is a diagram showing the characteristics of the specimens used in Example 4.

### [DESCRIPTION OF EMBODIMENTS]

Hereinafter, embodiments for carrying out the method for evaluating drug resistance of a microorganism according to the present invention will be described. The method for evaluating drug resistance of a microorganism according to the present embodiment comprises an extraction step of performing an operation to obtain an extract containing a drug-digested enzyme that can be produced by the microorganism from a sample containing the microorganism, a mixing step of mixing the extract and a drug, an analysis step of complexing the mixture obtained by the mixing step to mass spectrometry, and an evaluation step of determining whether the microorganism is resistant to the drug by detecting the presence or absence of a peak derived from a degradation product of the drug from the mass spectrum obtained in the analysis step.

### (Microorganism)

The microorganism whose resistance to a drug is to be evaluated is typically a bacterium. As for bacteria, both Gram-positive bacteria and Gram-negative bacteria can be evaluation targets of the present invention.

Examples of Gram-positive bacteria include the following: Listeria monocytogenes, Listeria welshimeri, Staphylococcus aureus, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Mycobacterium tuberculosis, Mycobacterium bovis, Mycobacterium leprae, Mycobacterium ulcerans, Mycobacterium kansasii, Mycobacterium avium, Mycobacterium paratuberculosis, Mycobacterium scrofulaceum, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroides, and Rhodococcus equi.

Examples of Gram-negative bacteria include the following: Neisseria gonorrhoeae, Neisseria meningitidis, Moraxella catarrhalis, Haemophilus influenzae, Klebsiella pneumoniae, Legionella pneumophila, Pseudomonas aeruginosa, Pseudomonas putida, Escherichia coli, Proteus mirabilis, Enterobacter cloacae, Serratia marcescens, Helicobacter pylori, Salmonella enteritidis, and Salmonella typhi.

### (Sample)

The sample contains a microorganism. The sample is, for example, a biological specimen (specimen) from a human or animal suffering from an infectious disease, and examples include urine, whole blood, serum, plasma, cerebrospinal fluid, pharyngeal mucus, sputum, or feces. From the viewpoint of ease of operation in the extraction step described later, the sample is preferably a liquid sample such as urine, whole blood, serum, plasma, and cerebrospinal fluid. Furthermore, from the viewpoint of containing a relatively large amount of microorganisms, the sample is more preferably urine.

### (Extraction Step)

In the extraction step, an operation is performed to obtain an extract containing a drug-digested enzyme that can be produced by the microorganism from a sample containing the microorganism. FIG. 1 is a flowchart showing the procedure of operations in the extraction step.

In the extraction step, first, a sample solution containing microorganisms is prepared (step S101). For example, the sample solution is prepared by adding saline solution or a liquid medium (L-broth) to the sample. The concentration of microorganisms in the sample solution is preferably 10³ CFU/mL or higher, more preferably 10⁵ CFU/mL, and particularly preferably 10⁶ CFU/mL. When the concentration of microorganisms in the sample solution is 10³ CFU/mL or higher, the presence or absence of drug resistance can be determined with high accuracy. Further, the concentration of microorganisms in the sample solution is preferably 10⁸ CFU/mL or lower. When preparing the sample solution, it is good to adjust the concentration of microorganisms to be within the above range. For example, the microorganisms contained in the sample solution may be cultured. Alternatively, the microorganisms contained in the sample may be isolated and cultured in advance, and a sample solution may be prepared by adding saline solution or the like to the isolated and cultured microorganisms to adjust the microorganism concentration. The urine of patients with urinary tract infections contains a relatively large amount of bacteria, and the concentration of bacteria often falls within the above range. Therefore, when the sample is a urine specimen, the urine specimen may be used as the sample solution as it is, without culturing the bacteria contained in the sample.

Next, the sample solution obtained in step S101 is centrifuged, and the supernatant is removed to obtain a precipitate (step S102). Then, a washing solution is added to the precipitate obtained in step S102, followed by centrifugation, and the supernatant is removed to obtain a precipitate. This washing operation is repeated twice (step S103). As the washing solution, phosphate-buffered saline (PBS) or the like can be used.

Subsequently, a surfactant or an acid is added to the precipitate obtained in step S103, held for a predetermined time, and then centrifuged to obtain a supernatant (step S104). As the surfactant, a nonionic surfactant or an amphoteric surfactant is preferable from the viewpoint of preventing denaturation of the enzymes contained in the microorganism. For example, BugBuster^{®} Protein Extraction Reagent (manufactured by Merck), which is a protein extraction reagent containing a surfactant, can be used. As the acid, for example, formic acid can be used.

When a solution containing a surfactant is used in step S104, the surfactant is removed from the supernatant obtained in step S104 (step S105). The removal of the surfactant can be performed by bringing the supernatant obtained in step S104 into contact with a resin that adsorbs the surfactant. For example, it can be performed by transferring the supernatant obtained in step S104 to a spin column filled with a resin that adsorbs the surfactant and centrifuging it. When an acid is used in step S104, it is not necessary to perform the process of step S105. An extract solution is obtained by the operation of step S104 (when using an acid) or step S105 (when using a surfactant).

Since the precipitate obtained in step S103 contains microorganisms that have undergone a washing operation, a portion of the precipitate may be used to prepare a sample for identification to be used for the identification of the microorganism by MALDI-MS. By doing so, both a sample for evaluation to be used for evaluating the drug resistance of the microorganism and a sample for identification to be used for identifying the microorganism can be obtained from a single sample.

### (Mixing Step)

In the mixing step, the extract solution prepared by the above-described extraction step and a drug are mixed. When the microorganism contained in the sample is a bacterium, the drug is a so-called antimicrobial agent.

Examples of antimicrobial agents include the following: penicillin-based antimicrobial agents such as Ampicillin (ABPC) and Piperacillin (PIPC); combination penicillin-based antimicrobial agents such as Sulbactam/Ampicillin (SBT/ABPC), Sulbactam/Cefoperazone (SBT/CPZ), and Tazobactam/Piperacillin (TAZ/PIPC); cephalosporin-based antimicrobial agents such as Cefazolin (CEZ), Cefotiam (CTM), Cefotaxime (CTX), Ceftazidime (CAZ), Cefpodoxime (CPDX), Ceftriaxone (CTRX), Cefditoren (CDTR), Cefpirome (CPR), and Cefepime (CFPM); cephamycin-based antimicrobial agents such as Cefmetazole (CMZ); oxacephem-based antimicrobial agents such as Latamoxef (LMOX); monobactam-based antimicrobial agents such as Aztreonam (AZT); carbapenem-based antimicrobial agents such as Imipenem (IPM) and Meropenem (MEPM); aminoglycosides such as Amikacin (AMK) and Gentamicin (GM); new quinolone-based antimicrobial agents such as Levofloxacin (LVFX) and Ciprofloxacin (CPFX); tetracycline-based antimicrobial agents such as Minomycin (MINO); and the combination agent Sulfamethoxazole-Trimethoprim (ST).

The method for mixing the extract solution and the drug is not particularly limited. For example, commercially available microplates in which various antimicrobial agents are dispensed and dried in wells can be used, and the extract solution and the drug may be mixed by adding the extract solution to the wells of such a microplate.

In the mixing step, the mixture of the extract solution and the drug is held at a predetermined temperature for a predetermined time under aerobic conditions. The temperature for holding the mixture is preferably a temperature at which the drug-digested enzyme that may be contained in the extract solution can easily work, for example, 35°C or higher and 40°C or lower. Further, the time for holding the mixture is preferably 5 minutes or longer, and more preferably 15 minutes or longer, from the viewpoint that the drug-digested enzyme that may be contained in the extract solution can sufficiently act on the drug. Further, the time for holding the mixture is preferably 30 minutes or shorter from the viewpoint that the evaluation of drug resistance can be performed rapidly.

### (Analysis Step)

In the analysis step, the mixture obtained by the above-described mixing step is subjected to mass spectrometry. The mass spectrometer is not particularly limited, and for example, MALDI-MS or LC-MS can be used. When using MALDI-MS as the mass spectrometer, a sample for MALDI mass spectrometry is prepared by dropping the mixture obtained by the mixing step onto a sample plate of the MALDI-MS and drying it, and then layering a matrix solution on top of it and drying it. As the matrix substance contained in the matrix solution, known substances can be used. An example of a matrix substance is α-Cyano-4-hydroxycinnamate (HCCA).

As the MALDI-MS, it is preferable to use a MALDI-TOFMS, which is a combination of a MALDI ion source and a time-of-flight mass spectrometer (TOFMS). In the identification of microorganisms by mass spectrometry, relatively high-mass molecules such as proteins, which are constituent components of the microorganisms, are analyzed. On the other hand, in the evaluation of drug resistance, relatively low-mass molecules such as the drug or degradation products of the drug are analyzed. Since MALDI-TOFMS has a wide range of measurable mass-to-charge ratios, if MALDI-TOFMS is used as the mass spectrometer, both the evaluation of drug resistance and the identification of the microorganism can be performed with a single apparatus.

### (Evaluation Step)

In the evaluation step, it is determined whether the microorganism is resistant to the drug by detecting the presence or absence of a peak derived from a degradation product of the drug from the mass spectrum obtained by mass spectrometry. If a peak derived from a degradation product of the drug is detected, it can be determined that the microorganism being evaluated is resistant to the drug. Alternatively, the presence or absence of drug resistance may be determined based on the peak intensities by extracting a peak derived from the drug and a peak derived from the degradation product of the drug from the mass spectrum obtained by mass spectrometry. For example, it may be determined that the microorganism is resistant if the value obtained by dividing the peak intensity of the peak derived from the degradation product of the drug by the sum of the peak intensity of the peak derived from the drug and the peak intensity of the peak derived from the drug degradation product exceeds a predetermined threshold.

### (Kit for Evaluating Microbial Drug Resistance)

A kit suitable for use in the method for evaluating microbial drug resistance according to the present embodiment is provided. For example, the kit for evaluating microbial drug resistance includes an extraction kit used to extract a drug-digested enzyme from a microorganism to obtain an extract, and a sample preparation kit for analysis used to prepare a sample for mass spectrometry using the extract. The extraction kit can include any reagent used in the above extraction step, any consumables other than reagents, a document describing the protocol for performing the above extraction step, and the like. For example, the extraction kit includes at least one of a surfactant and an acid. The sample preparation kit for analysis can include any reagent used in the above mixing step or analysis step, any consumables other than reagents, a document describing the protocol for performing the above mixing step or analysis step, and the like. For example, the sample preparation kit for analysis includes one or more antimicrobial agents. The sample preparation kit for analysis may include a microplate having compartments in which one or more antimicrobial agents are respectively contained. By using such a kit, the drug resistance of a microorganism can be evaluated more efficiently.

Hereinafter, the method for evaluating drug resistance of a microorganism according to the present invention will be described by some examples, but these are merely examples, and the present invention is not limited thereto.

The test strains and antimicrobial agents used in the following Experimental Examples and Examples are as follows.

### (Test Strains)

· E. coli (NCTC 13462): ESBL (Extended Spectrum β-Lactamase)-producing strain
· E. coli (ATCC 25922): Susceptible strain
(Antimicrobial Agents)
· Cefotaxime (CTX)
· Cefpodoxime (CPDX)
· Cefpirome (CPR)
· Cefpirome-Clavulanate (CPR/CLA)

### (Experimental Example 1)

### <Measurement of Antimicrobial Degradation Products by Microorganisms>

Using saline solution as a dispersion medium for the test strains, a suspension of the test strain adjusted to a turbidity of 1 McFarland unit was added at 100 µL to the wells of a dry plate 'Eiken' DPD-1 (manufactured by Eiken Chemical Co., Ltd.) containing CTX: 32 µg/mL and CPDX: 32 µg/mL. After culturing at 35°C under aerobic conditions for 30 minutes, 3 µL of the sample supernatant was applied to the target plate of a mass spectrometer and dried. Then, 1 µL of a matrix solution was layered on top and allowed to air dry to prepare a sample for analysis. The matrix solution was prepared by dissolving α-Cyano-4-hydroxycinnamate (HCCA) in 250 µL of a mixed solution of 50% Acetonitrile, 49.9% HPLC-grade water, and 0.1% Trifluoroacetic acid. A similar operation was performed by replacing the test strain suspension with saline solution to prepare an analysis sample (control sample). The mass spectrometer used was a MALDI-TOFMS (MALDI Biotyper^{™}, manufactured by Bruker Daltonics K.K.).

FIG. 2(a) is the mass spectrum when saline solution was brought into contact with CTX, FIG. 2(b) is the mass spectrum when a bacterial suspension of a susceptible strain was brought into contact with CTX, and FIG. 2(c) is the mass spectrum when a bacterial suspension of an ESBL-producing strain was brought into contact with CTX. In FIG. 2, for convenience, the horizontal axis of the mass spectrum is shown in mass (Da), but this is synonymous with the mass-to-charge ratio (m/z) when the charge number is 1. The same applies to the mass spectra shown in the subsequent figures. In all of FIG. 2(a), FIG. 2(b), and FIG. 2(c), a molecular ion peak of the matrix ([2M+H]+, where M is a matrix molecule and H is a hydrogen atom) was observed at 379.1 Da. In FIG. 2(a), peaks were observed at 455.9 Da and 477.8 Da, and in FIG. 2(b), peaks were observed at 456.0 Da and 478.0 Da. On the other hand, in FIG. 2(c), peaks were observed at 370.1 Da, 414.0 Da, and 455.9 Da. Between the peak observed at 370.1 Da and the peak observed at 414.0 Da, the peak observed at 370.1 Da had a higher peak intensity.

FIG. 3(a) is the mass spectrum when saline solution was brought into contact with CPDX, FIG. 3(b) is the mass spectrum when a bacterial suspension of a susceptible strain was brought into contact with CPDX, and FIG. 3(c) is the mass spectrum when a bacterial suspension of an ESBL-producing strain was brought into contact with CPDX. In all of FIG. 3(a), FIG. 3(b), and FIG. 3(c), a molecular ion peak of the matrix ([2M+H]+) was observed at 379.1 Da. In FIG. 3(a) and FIG. 3(b), peaks were observed at 428.0 Da and 450.0 Da. On the other hand, in FIG. 3(c), peaks were observed at 370.1 Da, 414.0 Da, 428.0 Da, and 450.0 Da. Between the peak observed at 370.1 Da and the peak observed at 414.0 Da, the peak observed at 370.1 Da had a higher peak intensity.

The peaks observed at 456 Da and 478 Da in the mass spectrum shown in FIG. 2 are thought to be derived from CTX, which exists as a carboxylate anion in saline solution, existing as a proton adduct (456 Da) or a sodium ion adduct (478 Da) in the ionization process. On the other hand, the peaks observed at 370 Da and 414 Da are thought to be derived from a degradation product (414 Da) generated after CTX undergoes hydrolysis by β-lactamase followed by a condensation cyclization reaction, and a degradation product (370 Da) generated when that degradation product further undergoes a decarboxylation reaction. Similarly, the peaks observed at 370 Da and 414 Da in the mass spectrum shown in FIG. 3 are thought to be derived from degradation products after CPDX is hydrolyzed by β-lactamase.

From the results of Experimental Example 1, in the following Examples 2 to 4, in the evaluation of resistance to CTX, the peaks observed at 456 Da and 478 Da were designated as non-hydrolyzed product-derived peaks, and the peak observed at 370 Da was designated as a hydrolyzed product-derived peak. Further, in the evaluation of resistance to CPDX, the peaks observed at 428 Da and 450 Da were designated as non-hydrolyzed product-derived peaks, and the peak observed at 370 Da was designated as a hydrolyzed product-derived peak.

### [EXAMPLE 1]

In Experimental Example 1, a suspension of the test strain was added to each antimicrobial agent, but in Example 1, an extract obtained from the suspension of the test strain was added to each antimicrobial agent, and the measurement of antimicrobial degradation products was performed.

### <Extraction of Drug-Digested Enzyme>

Using saline solution as a dispersion medium for the test strains, 1 mL of a suspension of the test strain adjusted to a turbidity of 1 McFarland unit was centrifuged at 14,000 rpm for 10 minutes. After removing the supernatant, the precipitate was washed with 1 mL of PBS, and the centrifugation step was repeated twice. After removing the supernatant, 100 µL of the protein extraction reagent BugBuster^{™} Protein Extraction Reagent (manufactured by Merck) was added to the precipitate to obtain a solution containing the precipitate and the reagent. The solution was allowed to stand for 10 minutes, then centrifuged at 14,000 rpm for 10 minutes, and the surfactant components were removed from the supernatant using Pierce Detergent Removal Spin Columns (manufactured by Thermo Scientific, Cat. No. 87777) to obtain an extract.

### <Measurement of Antimicrobial Degradation Products by Drug-Digested Enzyme>

50 µL of the extract obtained by the above extraction step was added to the CPR: 16 µg/mL well of a dry plate 'Eiken' DPE-1CPR (manufactured by Eiken Chemical Co., Ltd.). After incubation at 35°C under aerobic conditions for 30 minutes, 3 µL of the sample supernatant was applied to the target plate of a mass spectrometer and dried. Then, 1 µL of a matrix solution was layered on top and allowed to air dry to prepare a sample for analysis. A similar process was performed by replacing the extract with saline solution to prepare an analysis sample (control sample). The matrix solution and mass spectrometer used were the same as those used in Experimental Example 1.

### <Results>

FIG. 4(a) is the mass spectrum when saline solution was brought into contact with CPR, FIG. 4(b) is the mass spectrum when an extract from a susceptible strain was brought into contact with CPR, and FIG. 4(c) is the mass spectrum when an extract from an ESBL-producing strain was brought into contact with CPR. In all of FIG. 4(a), FIG. 4(b), and FIG. 4(c), a molecular ion peak of the matrix ([2M+H]⁺) was observed at 379.0 Da or 379.1 Da. In FIG. 4(a), peaks derived from non-hydrolyzed products of CPR were observed at 396.1 Da and 514.9 Da, and in FIG. 4(b), at 396.0 Da and 514.9 Da. On the other hand, in FIG. 4(c), while peaks derived from non-hydrolyzed products of CPR were observed at 396.1 Da and 514.9 Da, peaks derived from hydrolyzed products of CPR were also observed at 323.8 Da and 369.9 Da. Between the peak observed at 323.8 Da and the peak observed at 369.9 Da, the peak observed at 369.9 Da had a higher peak intensity.

From the results of Example 1, it was found that even when an extract is brought into contact with an antimicrobial agent, peaks derived from degradation products of the antimicrobial agent (324 Da and 370 Da) are observed, and susceptible strains and ESBL-producing strains can be distinguished. In the following Examples 2 to 4, in the evaluation of resistance to CPR, the peaks observed at 396 Da and 515 Da were designated as non-hydrolyzed product-derived peaks, and the peak observed at 370 Da was designated as a hydrolyzed product-derived peak.

### [EXAMPLE 2]

### [Study of Contact Time between Antimicrobial Agent and Extract]

### <Measurement of Antimicrobial Degradation Products by Drug-Digested Enzyme>

An extract obtained by the same extraction process as in Example 1 was added at 100 µL to the CTX: 32 µg/mL and CPDX: 32 µg/mL wells of a dry plate 'Eiken' DPE-1, and at 50 µL to the CPR: 16 µg/mL well. After incubation at 35°C under aerobic conditions for 5 minutes, 15 minutes, or 30 minutes, 3 µL of the sample supernatant was applied to the target plate of a mass spectrometer and dried. Then, 1 µL of a matrix solution was layered on top and allowed to air dry to prepare a sample for analysis. The matrix solution and mass spectrometer used were the same as those used in Experimental Example 1.

The measurement results were expressed as the value (Ihydro/Itotal) obtained by dividing the peak intensity of the hydrolyzed product-derived peak of each antimicrobial agent by the sum of the peak intensity of the non-hydrolyzed product-derived peak and the peak intensity of the hydrolyzed product-derived peak. For example, when the antimicrobial agent is CTX, if the peak intensities of the peaks observed at 370 Da, 456 Da, and 478 Da are I₃₇₀, I₄₅₆, and I₄₇₈, respectively, then Ihydro/Itotal = I₃₇₀ / (I₃₇₀ + I₄₅₆ + I₄₇₈). A larger value of Ihydro/Itotal means a higher proportion of hydrolyzed product in the sample.

### <Results>

FIG. 5(a) shows the Ihydro/Itotal values when an extract from a susceptible strain was brought into contact with various antimicrobial agents for a predetermined time, and FIG. 5(b) shows the Ihydro/Itotal values when an extract from an ESBL-producing strain was brought into contact with various antimicrobial agents for a predetermined time. When the antimicrobial agent was CTX, the Ihydro/Itotal (I₃₇₀/(I(₃₇₀₊₄₅₆₊₄₇₈))) for the susceptible strain was 0.07 to 0.09 regardless of the contact time, and no effect of contact time was observed. On the other hand, for the ESBL-producing strain, the Ihydro/Itotal was 0.73 with 5 minutes of contact, reached the highest value of 0.92 with 15 minutes of contact, but decreased slightly to 0.87 with 30 minutes of contact. When the antimicrobial agent was CPDX, the Ihydro/Itotal (I₃₇₀/(I₍₃₇₀₊₄₂₈₊₄₅₀₎)) for the susceptible strain was 0.05 to 0.06 regardless of the contact time, and no effect of contact time was observed. On the other hand, for the ESBL-producing strain, the Ihydro/Itotal was 0.38 with 5 minutes of contact, reached the highest value of 0.56 with 15 minutes of contact, but decreased slightly to 0.49 with 30 minutes of contact. When the antimicrobial agent was CPR, the Ihydro/Itotal (I₃₇₀/(I₍₃₇₀₊₃₉₆₊₅₁₅₎)) for the susceptible strain was 0.12 to 0.16 regardless of the contact time, and no effect of contact time was observed. On the other hand, for the ESBL-producing strain, the Ihydro/Itotal was 0.19 with 5 minutes of contact, which was similar to the susceptible strain, but as the contact time increased to 15 minutes and 30 minutes, the Ihydro/Itotal increased in a contact time-dependent manner to 0.48 and 0.72, respectively.

From the above results, it was found that for CTX and CPDX, susceptible strains and ESBL-producing strains can be distinguished by bringing the antimicrobial agent and the extract into contact for 5 minutes or longer. Further, for CPR, it was found that susceptible strains and ESBL-producing strains can be distinguished by bringing the antimicrobial agent and the extract into contact for 15 minutes or longer.

### [EXAMPLE 3]

### [Study of Lower Limit of Detection]

### <Extraction of Drug-Digested Enzyme>

An extract was obtained in the same manner as in Example 1, except that a urine specimen from a healthy subject was used as the dispersion medium for the test strains, and suspensions of the test strains adjusted to various bacterial concentrations (turbidity) were used.

### <Measurement of Antimicrobial Degradation Products by Drug-Digested Enzyme>

Antimicrobial degradation products were measured in the same manner as in Example 2.

### <Results>

FIG. 6 shows the Ihydro/Itotal values when measuring the degradation products of CTX, using a suspension of the test strain adjusted to various bacterial concentrations in the extraction of the drug-digested enzyme. FIG. 6(a) and FIG. 6(b) show the Ihydro/Itotal values when the contact time between the extract and the antimicrobial agent was 15 minutes and 30 minutes, respectively. Also, the values for the susceptible strain are indicated by white circles, and the values for the ESBL-producing strain are indicated by black circles (the same applies to FIG. 7 and FIG. 8 hereinafter). As shown in FIG. 6(a), when the contact time was 15 minutes, the Ihydro/Itotal for the susceptible strain was 0.1 or less regardless of the bacterial concentration. On the other hand, for the ESBL-producing strain, the Ihydro/Itotal value increased with the increase in bacterial concentration, and was 0.18 at 10⁵ CFU/mL, 0.60 at 10⁶ CFU/mL, and 0.92 at 10⁷ CFU/mL. Also, as shown in FIG. 6(b), when the contact time was 30 minutes, the Ihydro/Itotal for the susceptible strain was about 0.1 regardless of the bacterial concentration. On the other hand, for the ESBL-producing strain, it was 0.51 at 10⁵ CFU/mL, 0.85 at 10⁶ CFU/mL, and 0.98 at 10⁷ CFU/mL.

FIG. 7 shows the Ihydro/Itotal values when measuring the degradation products of CPDX, using a suspension of the test strain adjusted to various bacterial concentrations in the extraction of the drug-digested enzyme. As shown in FIG. 7(a), when the contact time was 15 minutes, the Ihydro/Itotal for the susceptible strain was 0.1 or less regardless of the bacterial concentration. On the other hand, for the ESBL-producing strain, the Ihydro/Itotal value increased with the increase in bacterial concentration, and was 0.20 at 10⁶ CFU/mL and 0.67 at 10⁷ CFU/mL. Also, as shown in FIG. 7(b), when the contact time was 30 minutes, the Ihydro/Itotal for the susceptible strain was 0.1 or less regardless of the bacterial concentration. On the other hand, for the ESBL-producing strain, it was 0.27 at 10⁵ CFU/mL, 0.65 at 10⁶ CFU/mL, and 0.95 at 10⁷ CFU/mL.

FIG. 8 shows the Ihydro/Itotal values when measuring the degradation products of CPR, using a suspension of the test strain adjusted to various bacterial concentrations in the extraction of the drug-digested enzyme. As shown in FIG. 8(a), when the contact time was 15 minutes, the Ihydro/Itotal for the susceptible strain was about 0.1 regardless of the bacterial concentration. On the other hand, for the ESBL-producing strain, the Ihydro/Itotal value increased with the increase in bacterial concentration, and was 0.20 at 10⁵-10⁶ CFU/mL and 0.45 at 10⁷ CFU/mL. Also, as shown in FIG. 8(b), when the contact time was 30 minutes, the Ihydro/Itotal for the susceptible strain was about 0.1 regardless of the bacterial concentration. On the other hand, for the ESBL-producing strain, it was 0.26 at 10⁵ CFU/mL, 0.32 at 10⁶ CFU/mL, and 0.86 at 10⁷ CFU/mL.

From the above results, it was found that when the extract and the antimicrobial agent were brought into contact for 15 minutes, susceptible strains and ESBL-producing strains could be distinguished if the bacterial concentration was 10⁶ CFU/mL or higher. Also, when the extract and the antimicrobial agent were brought into contact for 30 minutes, it was found that susceptible strains and ESBL-producing strains could be distinguished if the bacterial concentration was 10⁵ CFU/mL or higher.

### [EXAMPLE 4]

### [Study Using Clinical Specimens]

### <Extraction of Drug-Digested Enzyme>

10 mL of urine specimens (15 specimens) suspected of urinary tract infection from clinical practice, in which Gram-negative bacilli resembling Enterobacteriaceae were detected by Gram staining, were centrifuged at 3,400 rpm for 10 minutes, and the supernatant was removed. From the PBS washing step onwards, an extract was obtained in the same manner as in Example 1.

### <Measurement of Antimicrobial Degradation Products by Drug-Digested Enzyme>

In addition to CTX, CPDX, and CPR, the antimicrobial degradation products were measured in the same manner as in Example 3, except that 25 µL of the extract was added to the CPR/CLA (Cefpirome-Clavulanate): 4 µg/mL / 4 µg/mL well of the dry plate.

### <Results>

The characteristics of the 15 specimens used in this example are shown in FIG. 10. Among the 15 specimens, 5 specimens (E-1 to E-5) possessed the blaCTX-M type gene (a gene encoding β-lactamase), and E. coli was detected from all of these specimens. Among the 10 specimens (S-1 to S-10) that did not possess the blaCTX-M type gene, E. coli was detected from 5 specimens (S-1 to S-4, S-6), K. pneumoniae from 2 specimens (S-5, S-9), Morganella morganii from 1 specimen (S-7), and Enterobacter cloacae from 1 specimen (S-8). In addition, there was one specimen in which multiple bacterial species, E. coli and K. pneumoniae, were detected (S-10).

FIG. 9 shows the Ihydro/Itotal values when the extract from the urine specimens was brought into contact with various antimicrobial agents. The white circles indicate the values for the specimens containing susceptible strains (strains not possessing the blaCTX-M type gene) (S1 to S10 shown in FIG. 10), and the black circles indicate the values for the specimens containing ESBL-producing strains (strains possessing the blaCTX-M type gene) (E1 to E5 shown in FIG. 10). As shown in FIG. 9(a), in the 10 specimens containing susceptible strains, even when the extract and the antimicrobial agent were brought into contact for 15 minutes, the Ihydro/Itotal values were 0.09±0.02 for CTX, 0.05±0.03 for CPDX, and 0.11±0.01 for CPR. On the other hand, in the 5 specimens containing ESBL-producing strains, when the extract and the antimicrobial agent were brought into contact for 15 minutes, the Ihydro/Itotal values were 0.96±0.02 for CTX, 0.98±0.02 for CPDX, and 0.87±0.09 for CPR. Also, as shown in FIG. 9(b), even when the extract and the antimicrobial agent were brought into contact for 30 minutes, the Ihydro/Itotal values were similar to the results of 15 minutes of contact. Also, the Ihydro/Itotal value of CPR in the presence of CLA (clavulanic acid) was about 0.11 with 15 minutes of contact and about 0.13 with 30 minutes of contact in all specimens. CLA (clavulanic acid) is a β-lactamase inhibitor, and it was shown that β-lactamase is inhibited by CLA, resulting in Ihydro/Itotal values similar to those of susceptible strains. Furthermore, in the specimens where E. coli and E. faecalis were identified (E-5 and S-3 in FIG. 10), the signal intensity ratios for CTX, CPDX, and CPR were 1.0, 1.0, and 0.72 for the ESBL-producing strain, and 0.08, 0.05, and 0.11 for the susceptible strain, respectively, making it clear that ESBL-producing strains and susceptible strains can be distinguished in clinical specimens without being affected by coexisting bacteria.

### [Aspects]

It will be apparent to those skilled in the art that the exemplary embodiments described above are specific examples of the following aspects.

(Aspect 1) A method for evaluating drug resistance of a microorganism according to one aspect of the present invention comprises:
an extraction step of performing an operation to obtain an extract containing a drug-digested enzyme that can be produced by the microorganism from a sample containing the microorganism;
a mixing step of mixing the extract and a drug to obtain a mixture;
an analysis step of complexing the mixture to mass spectrometry; and
an evaluation step of determining whether the microorganism is resistant to the drug by detecting the presence or absence of a peak derived from a degradation product of the drug from the mass spectrum obtained in the analysis step.

According to the method for evaluating drug resistance of a microorganism according to Aspect 1, the presence or absence of resistance of a microorganism to a drug can be evaluated rapidly.

(Aspect 2) In the method for evaluating drug resistance of a microorganism according to Aspect 1, the drug-digested enzyme can be β-lactamase.

According to the method for evaluating drug resistance of a microorganism according to Aspect 2, the presence or absence of resistance of a microorganism to a drug that is degraded by β-lactamase can be evaluated.

(Aspect 3) In the method for evaluating drug resistance of a microorganism according to Aspect 1 or 2, in the extraction step, the extract can be obtained by bringing the microorganism into contact with a surfactant.

According to the method for evaluating drug resistance of a microorganism according to Aspect 3, the operation for extracting the drug-digested enzyme can be performed while suppressing denaturation of the drug-digested enzyme.

(Aspect 4) **In** the method for evaluating drug resistance of a microorganism according to any one of Aspects 1 to 3, in the extraction step, the operation can be performed without culturing the microorganism.

According to the method for evaluating drug resistance of a microorganism according to Aspect 4, the presence or absence of resistance of a microorganism to a drug can be evaluated more rapidly.

(Aspect 5) **In** the method for evaluating drug resistance of a microorganism according to any one of Aspects 1 to 4, the sample can be urine.

According to the method for evaluating drug resistance of a microorganism according to Aspect 5, since a sample containing a relatively large amount of microorganisms can be obtained, the presence or absence of resistance of a microorganism to a drug can be evaluated without culturing the microorganism.

(Aspect 6) **In** the method for evaluating drug resistance of a microorganism according to any one of Aspects 1 to 5, the method may further comprise a step in the extraction step of collecting a separate of the microorganism from the sample, performing an operation to obtain the extract using a part of the separate, and preparing a sample for identification for identifying the microorganism contained in the sample using a part of the separate.

According to the method for evaluating drug resistance of a microorganism according to Aspect 6, both the evaluation of drug resistance of a microorganism and the identification of the microorganism can be performed from a single sample.

(Aspect 7) **In** the method for evaluating drug resistance of a microorganism according to any one of Aspects 1 to 6, the mass spectrometry may be matrix-assisted laser desorption/ionization mass spectrometry.

According to the method for evaluating drug resistance of a microorganism according to Aspect 7, both the evaluation of drug resistance of a microorganism and the identification of the microorganism can be performed with a single apparatus.

(Aspect 8) A kit for evaluating drug resistance of a microorganism according to one aspect of the present invention is a kit for performing the method for evaluating drug resistance of a microorganism according to any one of Aspects 1 to 7, which can contain a surfactant.

(Aspect 9) A kit for evaluating drug resistance of a microorganism according to one aspect of the present invention is a kit for performing the method for evaluating drug resistance of a microorganism according to any one of Aspects 1 to 7, which can contain one or more antimicrobial agents.

According to the kit for evaluating drug resistance of a microorganism according to Aspect 8 or 9, the drug resistance of a microorganism can be evaluated efficiently.

## Claims

1. A method for evaluating drug resistance of a microorganism, the method comprising:
an extraction step of performing an operation to obtain an extract containing a drug-digested enzyme that can be produced by the microorganism from a sample containing the microorganism;
a mixing step of mixing said extract and a drug to obtain a mixture;
an analysis step of complexing said mixture to mass spectrometry; and
an evaluation step of determining whether said microorganism is resistant to said drug by detecting a presence or absence of a peak derived from a degradation product of said drug from a mass spectrum obtained in said analysis step.

2. The method for evaluating drug resistance of a microorganism according to claim 1, wherein said drug-digested enzyme is β-lactamase.

3. The method for evaluating drug resistance of a microorganism according to claim 1 or 2, wherein in said extraction step, said operation is performed by bringing said microorganism into contact with a surfactant.

4. The method for evaluating drug resistance of a microorganism according to claim 1 or 2, wherein in said extraction step, said operation is performed without culturing said microorganism.

5. The method for evaluating drug resistance of a microorganism according to claim 1 or 2, wherein said sample is urine.

6. The method for evaluating drug resistance of a microorganism according to claim 1 or 2, further comprising a step in said extraction step of:
collecting a separate of said microorganism from said sample;
performing the operation to obtain said extract using a first part of said separate; and
preparing a sample for identification for identifying said microorganism contained in said sample using a second part of said separate.

7. The method for evaluating drug resistance of a microorganism according to claim 1 or 2, wherein said mass spectrometry is matrix-assisted laser desorption/ionization mass spectrometry.

8. A kit for evaluating drug resistance of a microorganism for performing the method according to claim 1 or 2, the kit comprising a surfactant.

9. A kit for evaluating drug resistance of a microorganism for performing the method according to claim 1 or 2, the kit comprising one or more antimicrobial agents.
